# FASCICULE DE BREVET EUROPEEN

(11) **EP 1 171 055 B1**
(45) Date de publication et mention de la délivrance du brevet: **14.01.2004**
(21) Numéro de dépôt: 00917143.0
(22) Date de dépôt: 06.04.2000
(51) Int. Cl.: A61F 2/00, A61B 17/04

(54) **DISPOSITIF DE TRAITEMENT DE PROLAPSUS PAR SUSPENSION VAGINALE**
BEHANDLUNGSVORRICHTUNG FÜR PROLAPSUS DURCH ABSTÜTZUNG DER VAGINA
DEVICE FOR TREATING A PROLAPSE BY VAGINAL SUSPENSION

(30) Priorité: 27.04.1999 FR 9905487
(43) Date de publication de la demande: 16.01.2002
(73) Titulaire: SOFRADIM PRODUCTION, 01600 Trévoux (FR)
(72) Inventeur: GASTON, Richard-Pierre, F-33200 Bordeaux-Cauderan (FR)
(74) Mandataire: Maureau, Philippe
(86) Numéro de dépôt international: PCT/FR2000/000874
(87) Numéro de publication internationale: WO 2000/064370

(56) Documents cités:
- EP-A- 0 248 544
- WO-A-97/13465
- WO-A-98/35632

## Description

La présente invention concerne un dispositif de traitement de prolapsus par suspension vaginale.

Les prolapsus génitaux, urinaires ou rectaux proviennent d'un relâchement des tissus soutenant les organes, ainsi que du périnée, qui provoquent chez la femme âgée une incontinence urinaire à l'effort.

Le traitement chirurgical de ces prolapsus implique de relier un ou plusieurs de ces organes (vessie, vagin-utérus, rectum) à des zones anatomiquement stables, notamment, côté antérieur, au niveau du ligament de Cooper, sur le bord postéro-supérieur du pubis ou, du côté postérieur, au niveau du promontoire, c'est-à-dire de l'angle antéro-supérieur du sacrum, en "suspendant" ces organes à ces zones anatomiquement stables.

Le traitement chirurgical des prolapsus est actuellement réalisé au moyen de sutures non résorbables ou de bandelettes de renfort.

Les sutures ont pour avantage d'être simples à mettre en place lors du traitement d'un prolapsus par voie ouverte, d'avoir un coût réduit et des possibilités d'utilisation étendues.

Elles ont toutefois pour inconvénient d'assurer une fixation ponctuelle et peu élastique du fil de suspension, qui est susceptible de provoquer un cisaillement des tissus sur lesquels elles sont mises en place, conduisant à une rupture de l'ancrage. Il en résulte que leur efficacité est modérée dans le temps. De plus, elles sont complexes à mettre en place lors du traitement des prolapsus par voie laparoscopique, compte tenu de la nécessité de faire de nombreux noeuds.

Les bandelettes ont pour avantages d'être simples à mettre en place quelle que soit la voie employée (voie ouverte ou laparoscopique), d'être efficaces et de permettre une répartition sur plusieurs points d'ancrage des contraintes qui sont exercées. Elles sont en outre susceptibles d'une intégration rapide à la paroi d'ancrage et aux tissus environnants, par réhabitation tissulaire.

Ces bandelettes ont toutefois pour inconvénient de devoir être vrillées lorsque les parois d'implantation respectives ne sont pas parallèles. La zone vrillée travaille dans des conditions guère favorables, en se déplaçant le long de la bande lors d'une tension, avec amincissement de cette dernière en son centre, rendant sa mise en place malaisée et accentuant l'effet de cisaillement. Une bande en matériau relativement rigide, tel qu'en polypropylène monofilament, présente des bords pouvant être traumatisants pour les tissus environnants, sur l'ensemble de la longueur de la bande.

Un dispositif de traitement de prolapsus est décrit dans le document WO 98/35632.

Le traitement d'un prolapsus par suspension vaginale est délicat, compte tenu de la relative fragilité de la paroi du vagin, et les dispositifs existant pour réaliser une telle suspension, particulièrement par voie laparoscopique, ne donnent pas parfaitement satisfaction.

La présente invention vise à remédier à cette lacune.

A cet effet, le dispositif qu'elle concerne comprend un ensemble formé par une pièce allongée en matériau souple ajouré, un fil de suture relié à une extrémité longitudinale de ladite pièce et une aiguille de suture reliée à ce fil ; la pièce présente une longueur telle qu'elle permet une suspension postérieure du vagin au promontoire, c'est-à-dire à la partie antéro-supérieure du sacrum ; cette pièce comprend, du côté de son extrémité reliée au fil de suture, (i) une portion distale présentant une largeur telle qu'elle peut recouvrir au moins une large partie de la moitié postérieure de la paroi du vagin et qu'elle peut être suturée très latéralement, donc de façon non transfixiante dans le paravagin, et (ii) une découpe arrondie aménagée dans son bord latéral d'extrémité distale, cette découpe ayant des dimensions telles qu'elle permet l'engagement de la pièce autour de la base de la paroi du vagin, sur au moins une large partie de la moitié postérieure de cette paroi, tout en laissant le rectum suffisamment à distance pour ne pas risquer la compression de celui-ci ; le fil de suture est relié à la pièce de manière décalée latéralement par rapport à ladite découpe.

La mise en place du dispositif selon l'invention est réalisée comme suit. Après installation de trocarts optiques et opératoires, une incision arciforme de la face antérieure du cul-de-sac de Douglas (vagino-rectal) est réalisée, puis il est procédé à une dissection du fascia pré-rectal, se poursuivant latéralement jusqu'aux faisceaux postérieurs des muscles releveurs, de part et d'autre du rectum. Une valve vaginale peut être aménagée pour faciliter la dissection jusqu'au plancher pelvien au niveau du ligament sacrosciatique.

L'aiguille est descendue entre le vagin et le rectum à partir du cul-de-sac de Douglas, jusqu'au niveau du plancher pelvien, puis est engagée latéralement au travers des muscles releveurs de manière à assurer un point de coulissement solide du fil ; ce dernier est ensuite coulissé par traction sur l'aiguille, afin de provoquer la descente de la pièce en matériau ajouré dans l'espace disséqué, jusqu'à ce que cette pièce se positionne contre la face postérieure du vagin. La découpe que présente la portion distale de cette pièce permet un large engagement de cette portion autour de la base du vagin sans compression du rectum, et la largeur de cette même portion permet un contact de la pièce avec la face postérieure du vagin selon une large surface.

La pièce est ensuite fixée d'un coté sur le plancher pelvien au moyen du fil précité sur la face postérieure du vagin par des points de suture successifs jusqu'au ligament utéro-sacré à l'aide de ce même fil. Les mêmes gestes sont répétés avec une deuxième ligature de 25 cm de l'autre côté à partir de l'autre extrémité distale de la pièce.

La pièce est alors fixée au promontoire par son extrémité proximale, pour réaliser la suspension.

Cette pièce permet ainsi, de par sa forme, une fixation distale non pas à la paroi du vagin mais aux muscles releveurs et aux ligaments utéro-sacrés, donc en des emplacements résistants, pour l'obtention d'une prise d'appui dans des conditions optimales.

La structure ajourée de la pièce permet de limiter au maximum les risques de réaction tissulaire exacerbée (fibrose) et d'obtenir une intégration rapide aux tissus, par croissance de ces derniers au travers des mailles.

De préférence, le dispositif selon l'invention comprend non seulement l'ensemble pièce en matériau ajouré - fil de suture - aiguille de suture précité, destiné à être mis en place au niveau de la face postérieure du vagin, mais également un deuxième ensemble formé d'une pièce en matériau ajouré, d'un fil de suture et d'une aiguille de suture, destiné à être mis en place au niveau de la face antérieure du vagin, en complément du premier ensemble.

Ce deuxième ensemble comprend une pièce allongée en matériau souple ajouré, un fil de suture relié à une extrémité longitudinale de la pièce et une aiguille de suture reliée à ce fil ; ladite pièce présente une longueur telle qu'elle permet une suspension antérieure du vagin au promontoire, et comprend, du côté de son extrémité reliée audit fil, deux bords latéraux obliques convergeant l'un vers l'autre en direction de cette extrémité, de telle sorte que la pièce présente une portion allant se rétrécissant progressivement vers cette extrémité ; le fil de suture est relié à cette extrémité.

La mise en place de ce deuxième ensemble est réalisée comme suit. Il est procédé à une dissection de la face antérieure du vagin au niveau du cul-de-sac vésico-vaginal, puis à une perforation du paravagin sous les annexes. L'aiguille de ce deuxième ensemble est engagée le long de la face antérieure du vagin et est engagée au travers d'un tissu, le plus près possible de la base de ce vagin, afin de constituer un point de coulissement solide du fil ; ce dernier est ensuite coulissé pour permettre la descente de la pièce le long de la face antérieure du vagin. La portion de cette pièce allant en se rétrécissant dans la direction distale correspond à la forme distale du cul-de-sac vésico-vaginal, ce qui permet l'engagement de ladite pièce le plus loin possible, pour une prise d'appui dans un tissu relativement épais et résistant.

Par un surjet à l'aide de ce même fil, la pièce en matériau ajouré est fixée le long de l'une de ses berges à la paroi latérale du vagin à hauteur de l'isthme utérin. L'autre berge est suturée de façon identique par un surjet à l'aide d'un deuxième fil de 25 cm de long. Afin d'éviter la transfixation du vagin tout en assurant un ancrage mécaniquement satisfaisant, la prise du tissu vaginal dans le surjet est large mais peu profonde.

La pièce en matériau ajouré est alors fendue longitudinalement à partir de son extrémité proximale, pour constituer deux brins qui sont engagés de part et d'autre de l'isthme utérin, sous les annexes, et qui sont fixés par leur portion proximale au promontoire, au même niveau que la pièce assurant la suspension postérieure.

Ce deuxième ensemble permet ainsi, à titre complémentaire, une suspension antérieure du vagin, et les suspensions antérieure et postérieure réalisées par les deux ensembles précités assurent conjointement un parfait traitement du prolapsus.

Ladite pièce du deuxième ensemble pourrait non pas être fendue par le praticien au moment de l'intervention mais être fendue dès la fabrication dudit ensemble.

Avantageusement, chaque ensemble pièce en matériau ajouré - fil de suture - aiguille de suture comprend un brin de fil de suture dont une extrémité est reliée à la pièce en matériau ajouré, à proximité immédiate de l'extrémité du fil de suture.

Ce brin facilite la réalisation du premier noeud de fixation de la pièce en matériau ajouré.

Selon une forme de réalisation simple de l'invention dans ce cas, le fil de suture est simplement relié à la pièce en matériau ajouré par nouage, et le brin précité est constitué par une portion du fil de suture se prolongeant au-delà du noeud.

La ou les pièces en matériau ajouré sont avantageusement réalisées en tricot de fils polyester multibrins à mailles quadrillées.

Ce matériau convient particulièrement à la mise en oeuvre de l'invention.

Avantageusement, chaque pièce est imprégnée de collagène, pour favoriser cette réhabitation tissulaire. Leur texture indémaillable permet une prise légère pour bénéficier de toute la largeur de la pièce. Leurs larges pores facilitent le passage de l'aiguille.

Le fil de suture est de type classique, et est notamment constitué par une tresse de fils en polyester. Il présente de préférence une longueur de l'ordre de 25 cm, nécessaire pour l'exécution d'un surjet ou de points séparés et suffisante pour ne pas être encombrante dans l'espace réduit qu'impose la laparoscopie.

L'aiguille de suture a, quant à elle, avantageusement une forme courbe suffisante pour ne pas être transfixiante lors de son introduction sur la paroi vaginale, et une section triangulaire pour faciliter son passage dans des tissus parfois épais. Elle est de préférence sertie sur l'extrémité du fil de suture.

Pour sa bonne compréhension, l'invention est à nouveau décrite ci-dessous en référence au dessin schématique annexé représentant, à titre d'exemple non limitatif, une forme de réalisation préférée du dispositif qu'elle concerne.
La figure 1 est une vue à plat d'un premier ensemble que comprend ce dispositif ;
la figure 2 est une vue à plat d'un deuxième ensemble que comprend ce dispositif ;
la figure 3 est une vue en coupe très simplifiée de la partie inférieure d'un tronc, avec ledit premier ensemble mis en place, et
la figure 4 est une vue similaire à la figure 3, avec lesdits premier et deuxième ensembles mis en place.

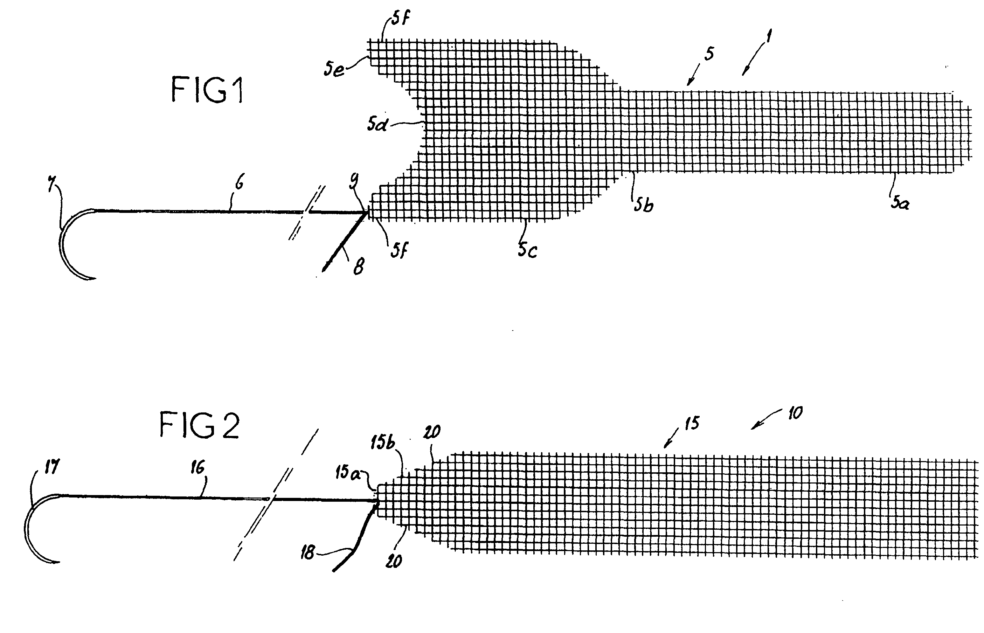

La figure 1 représente un ensemble 1 permettant de traiter un prolapsus par suspension postérieure du vagin 2 au promontoire 3, c'est-à-dire à l'angle antéro-supérieur du sacrum 4.

Cet ensemble 1 comprend :
- une pièce 5 en tissu de fils polyester multibrins à mailles carrées, ces mailles ayant environ deux millimètres de côté,
- un fil de suture 6,
- une aiguille de suture 7 et
- un brin de suture 8.

La pièce 5 présente une longueur telle qu'elle peut relier la base du vagin 2 au promontoire 3. Elle comprend une partie proximale 5a destinée à être reliée au promontoire 3 et une partie distale 5b destinée à être reliée, au niveau distal, à des tissus environnant la base du vagin 2.

La partie proximale 5a présente une largeur suffisante pour assurer la résistance de la pièce 5 aux efforts engendrés par la suspension.

La partie distale 5b présente une portion 5c élargie, dont la largeur est supérieure à la moitié postérieure de la paroi du vagin 2. Cette portion 5c comprend une découpe 5d dans son bord d'extrémité distale 5e, allant en s'évasant en direction de ce bord d'extrémité 5e, le rayon de cette découpe 5d correspondant sensiblement à celui d'un vagin 2 au niveau de sa base.

Le fil de suture 6 est constitué par une tresse de fils de polyester et est noué à l'une des parties saillantes 5f de la pièce 5 qui délimitent latéralement ladite découpe 5d. Ce fil 6 se prolonge au-delà du noeud 9 qui le relie à la pièce 5 pour constituer le brin de suture 8 précité.

L'aiguille 7 est sertie sur l'extrémité libre du fil 6 par son extrémité proximale et présente une forme courbe.

La figure 2 représente un deuxième ensemble 10, de même structure générale que l'ensemble 1, c'est-à-dire comprenant :
- une pièce 15 en tissu de fils polyester multibrins à mailles carrées, ces mailles ayant deux millimètres de côté,
- un fil de suture 16,
- une aiguille de suture 17, et
- un brin de suture 18.

La pièce 15 présente une longueur permettant la suspension de la paroi antérieure du vagin 2 au promontoire 3, et une largeur suffisante pour assurer sa résistance aux efforts engendrés par la suspension.

Cette pièce 15 présente en outre deux bords obliques 20 au niveau de sa partie distale 15b, qui convergent l'un vers l'autre en direction de l'extrémité distale 15a de la pièce 15. Cette partie distale 15b présente ainsi une largeur allant en se réduisant en direction de cette extrémité 15a.

Le fil de suture 16 est fixé au niveau de l'axe médian de la pièce 15, à la pointe de cette partie 15b, par nouage.

Ceci mis à part, le fil 16, l'aiguille 17 et le brin 18 sont en tous points similaires à ceux précités de l'ensemble 1.

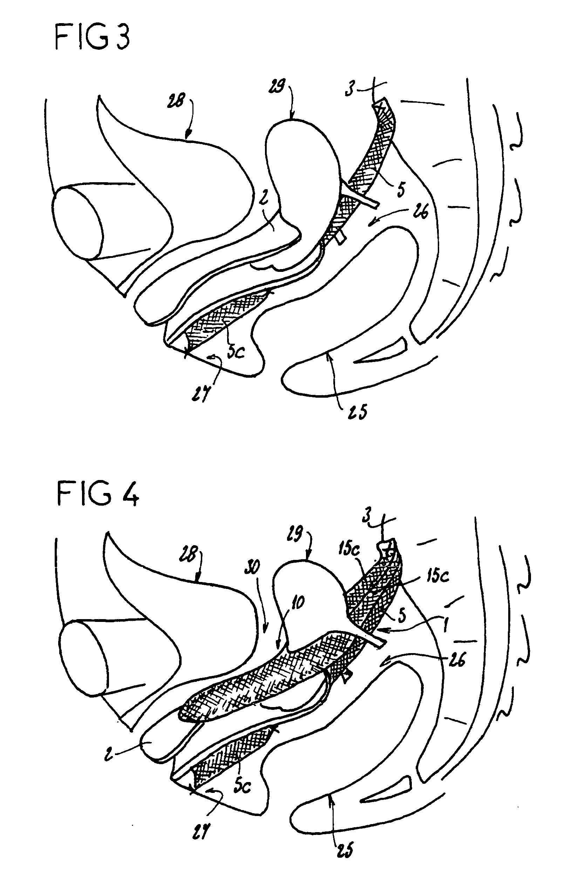

Comme le montre la figure 3, la pièce 5 est destinée à être engagée entre le vagin 2 et le rectum 25, à partir du cul-de-sac de Douglas 26, jusqu'au niveau du plancher pelvien 27, et à être fixée, au niveau distal, aux muscles releveurs.

La figure 4 montre que la pièce 15 est destinée à être engagée entre le vagin 2 et la vessie 28 et à être fixée au niveau distal à la paroi du vagin 2. Cette pièce 15 est en outre destinée à être fendue longitudinalement à partir de son bord proximal pour constituer deux brins 15c passant de part et d'autre de l'isthme de l'utérus 29.

Plus précisément, la mise en place du dispositif selon l'invention est réalisée comme suit. Après installation de trocarts optiques et opératoires, une incision arciforme de la face antérieure du cul-de-sac de Douglas 26 (vagino-rectal) est réalisée, puis il est procédé à une dissection du fascia pré-rectal, se poursuivant latéralement jusqu'aux faisceaux postérieurs des muscles releveurs de part et d'autre du rectum 25. Une valve vaginale peut être aménagée pour faciliter la dissection jusqu'au plancher pelvien 27 au niveau du ligament sacrosciatique.

L'aiguille 7 est descendue entre le vagin 2 et le rectum 25 à partir du cul-de-sac de Douglas 26, jusqu'au niveau du plancher pelvien 27, puis est engagée latéralement au travers d'un tissu de manière à assurer un point de coulissement solide du fil 6 ; ce dernier est ensuite coulissé par traction sur l'aiguille 7, afin de provoquer la descente de la pièce 5 dans l'espace disséqué le long du vagin 2, jusqu'à ce que cette pièce 5 se positionne contre la face postérieure du vagin 5. La découpe 5d permet un large engagement de la portion distale élargie 5c autour de la base du vagin 2, et la largeur de cette même portion 5c permet un contact de la pièce 5 avec la face postérieure du vagin selon une large surface.

La pièce 5 est ensuite fixée sur le plancher pelvien par suture réalisée au moyen du fil 6 et du brin 8, puis est fixée de part et d'autre du vagin 2, par des points de suture réalisés à l'aide de ce fil 6.

Elle est ensuite rabattue contre la face postérieure du vagin 2 et est fixée aux ligaments utéro-sacrés.

Il est alors procédé à une dissection de la face antérieure du vagin 2 au niveau du cul-de-sac vésico-vaginal 30, puis à une perforation du paravagin sous les annexes. L'aiguille 17 du deuxième ensemble est engagée le long de la face antérieure du vagin 2 et est engagée au travers d'un tissu, le plus près possible de la base de ce vagin, afin de constituer un point de coulissement solide du fil 16 ; ce dernier est ensuite coulissé pour permettre la descente de la pièce 15 le long de la face antérieure du vagin 2. La portion distale 15b de cette pièce 15 correspond à la forme distale du cul-de-sac vésico-vaginal 30, ce qui permet l'engagement de ladite pièce 15 le plus loin possible dans la direction distale.

La pièce 15 est ensuite fixée à la paroi du vagin 2, à hauteur de la portion distale de celui-ci, par un surjet ayant une prise large mais peu profonde dans le tissu vaginal.

La pièce 15 est alors fendue longitudinalement à partir de son extrémité proximale, pour constituer les deux brins 15c, et ceux-ci sont engagés de part et d'autre de l'isthme utérin, sous les annexes.

Ces deux brins 15c sont ensuite fixés par leur portion proximale au promontoire 3, pour réaliser la suspension vaginale.

L'invention fournit ainsi un dispositif de traitement de prolapsus par suspension vaginale qui remédie aux inconvénients des techniques antérieures, en permettant une fixation distale postérieure non pas à la paroi du vagin mais au plancher pelvien et aux ligaments utéro-sacrés, donc en des emplacements résistants, et, à titre complémentaire, une suspension distale antérieure prenant appui dans un tissu relativement épais et résistant.

## Revendications

1. Dispositif de traitement de prolapsus par suspension vaginale, comprenant un ensemble (1) formé par une pièce allongée (5) en matériau souple ajouré, un fil de suture (6) relié à une extrémité longitudinale de ladite pièce (5) et une aiguille de suture (7) reliée à ce fil (6) ; la pièce (5) présentant une longueur telle qu'elle permet une suspension postérieure du vagin (2) au promontoire (3), c'est-à-dire à la partie antéro-supérieure du sacrum ; cette pièce (5) comprenant, du côté de son extrémité reliée au fil de suture, (i) une portion distale (5c) présentant une largeur telle qu'elle peut recouvrir au moins une large partie de la moitié postérieure de la paroi du vagin (2) et qu'elle peut être suturée très latéralement, donc de façon non transfixiante dans le paravagin, et (ii) une découpe (5d) arrondie aménagée dans son bord latéral d'extrémité distale (5e), cette découpe (5d) ayant des dimensions telles qu'elle permet l'engagement de la pièce (5) autour de la base de la paroi du vagin (2), sur au moins une large partie de la moitié postérieure de cette paroi, tout en laissant le rectum suffisamment à distance pour ne pas risquer la compression de celui-ci ; le fil de suture (6) étant relié à la pièce (5) de manière décalée latéralement par rapport à ladite découpe (5d).

2. Dispositif selon la revendication 1, **caractérisé en ce qu'**il comprend un deuxième ensemble (10) formé d'une pièce en matériau ajouré (15), d'un fil de suture (16) et d'une aiguille de suture (17), destiné à être mis en place au niveau de la face antérieure du vagin (2), en complément du premier ensemble (1) ; ce deuxième ensemble comprend une pièce allongée (15) en matériau souple ajouré, un fil de suture (16) relié à une extrémité longitudinale de la pièce (15) et une aiguille de suture (17) reliée à ce fil (16) ; ladite pièce (15) présente une longueur telle qu'elle permet une suspension antérieure du vagin (2) au promontoire (3), et comprend, du côté de son extrémité (15e) reliée audit fil (16), deux bords latéraux obliques (20) convergeant l'un vers l'autre en direction de cette extrémité (15e), de telle sorte que la pièce (15) présente une portion (15b) allant se rétrécissant progressivement vers cette extrémité (15e) ; le fil de suture (16) est relié à cette extrémité (15e).

3. Dispositif selon la revendication 2, **caractérisé en ce que** ladite pièce (15) en matériau ajouré du deuxième ensemble (10) est fendue longitudinalement à partir de son extrémité proximale, pour constituer deux brins (15c) destinés à être engagés de part et d'autre de l'isthme utérin, sous les annexes, et à être fixés par leur portion proximale au promontoire (3).

4. Dispositif selon l'une des revendications 1 à 3, **caractérisé en ce que** chaque ensemble (1, 10) pièce en matériau ajouré - fil de suture - aiguille de suture comprend un brin de fil de suture (8, 18) dont une extrémité est reliée à la pièce (5, 15) en matériau ajouré, à proximité immédiate de l'extrémité du fil de suture (6, 16).

5. Dispositif selon la revendication 4, **caractérisé en ce que** le fil de suture (6, 16) est relié à la pièce (5, 15) en matériau ajouré par nouage, et **en ce que** le brin de suture (8, 18) est constitué par une portion du fil de suture (6, 16) se prolongeant au-delà du noeud (9).

6. Dispositif selon l'une des revendications 1 à 5, **caractérisé en ce que** chaque pièce (5, 15) en matériau ajouré est réalisée en tricot de fils polyester multibrins à mailles quadrillées.

7. Dispositif selon l'une des revendications 1 à 6, **caractérisé en ce que** chaque pièce (5, 15) est imprégnée de collagène.

8. Dispositif selon l'une des revendications 1 à 7, **caractérisé en ce que** le fil de suture (6, 16) est constitué par une tresse de fils en polyester.

9. Dispositif selon l'une des revendications 1 à 8, **caractérisé en ce que** le fil de suture (6, 16) présente une longueur de l'ordre de 25 cm.

10. Dispositif selon l'une des revendications 1 à 9, **caractérisé en ce que** l'aiguille de suture (7, 17) a une forme courbe suffisante pour ne pas être transfixiante lors de son introduction sur la paroi vaginale, et une section triangulaire pour faciliter son passage dans des tissus parfois épais.

## Claims

1. Device for treating a prolapse by vaginal suspension, comprising a unit (1) formed by an elongate part (5) of flexible open-worked material, a suture thread (6) connected to a longitudinal end of the said part (5), and a suture needle (7) connected to this thread (6); the part (5) having a length which is such that it permits posterior suspension of the vagina (2) at the promontory (3), that is to say at the antero-superior portion of the sacrum; this part (5) comprising, at its end connected to the suture thread, (i) a distal portion (5c) whose width is such that it can cover at least a wide area of the posterior half of the wall of the vagina (2) and it can be sutured very laterally, that is to say in a manner not transfixing the paravagina, and (ii) a rounded cut-out (5d) formed in its lateral edge at the distal end (5e), this cut-out (5d) having dimensions which are such that it permits engagement of the part (5) around the base of the wall of the vagina (2), over at least a wide area of the posterior half of this wall, while at the same time leaving the rectum at a sufficient distance so as not to risk compressing the latter; the suture thread (6) being connected to the part (5) in such a way that it is offset laterally in relation to the said cut-out (5d) .

2. Device according to Claim 1, **characterized in that** it comprises a second unit (10) formed by a part of open-worked material (15), a suture thread (16) and a suture needle (17), the said second unit (10) being intended to be placed at the level of the anterior surface of the vagina (2) to complement the first unit (1); this second unit comprises an elongate part (15) of flexible open-worked material, a suture thread (16) connected to a longitudinal end of the part (15), and a suture needle (17) connected to this thread (16); the said part (15) has a length which is such that it permits an anterior suspension of the vagina (2) at the promontory (3), and comprises, at its end (15e) connected to the said thread (16), two oblique lateral edges (20) converging towards each other in the direction of this end (15e) such that the part (15) has a portion (15b) which narrows gradually towards this end (15e); the suture thread (16) is connected to this end (15e).

3. Device according to Claim 2, **characterized in that** the said part (15) of open-worked material of the second unit (10) is slit longitudinally starting from its proximal end in order to constitute two strands (15c) intended to be engaged each side of the uterine isthmus, below the adnexa, and to be fixed via their proximal portion to the promontory (3).

4. Device according to one of Claims 1 to 3, **characterized in that** each unit (1, 10) consisting of part of open-worked material, suture thread and suture needle, comprises a strand of suture thread (8, 18), one end of which is connected to the part (5, 15) of open-worked material, in immediate proximity to the end of the suture thread (6, 16).

5. Device according to Claim 4, **characterized in that** the suture thread (6, 16) is connected to the part (5, 15) of open-worked material by knotting, and **in that** the suture strand (8, 18) consists of a portion of the suture thread (6, 16) continuing beyond the knot (9) .

6. The device according to one of Claims 1 to 5, **characterized in that** each part (5, 15) of open-worked material is formed as a knit of multifilament polyester yarns with square meshes.

7. Device according to one of Claims 1 to 6, **characterized in that** each part (5, 15) is impregnated with collagen.

8. Device according to one of Claims 1 to 7, **characterized in that** the suture thread (6, 16) consists of a braid of polyester yarns.

9. Device according to one of Claims 1 to 8, **characterized in that** the suture thread (6, 16) has a length of the order of 25 cm.

10. Device according to one of Claims 1 to 9, **characterized in that** the suture needle (7, 17) has a curved shape sufficient to prevent transfixing upon its introduction on the vaginal wall, and a triangular cross section to facilitate its passage in sometimes thick tissues.

## Patentansprüche

1. Vorrichtung zum Behandeln eines Prolaps durch Vaginalsuspension, mit einer Anordnung (1), die durch ein längliches Stück (5) aus einem durchbrochenen flexiblen Material, einen Nähfaden (6), der mit einem Längsende des Stücks (5) verbunden ist und eine Nähnadel (7), die mit dem Faden (6) verbunden ist, gebildet ist, wobei das Stück (5) dabei eine solche Länge aufweist, die eine hintere Suspension der Vagina (2) am promontorium (3), d.h. am vorderen oberen Abschnitt des Sakrums ermöglicht; wobei das Stück (5) auf der Seite seines Endes, das mit dem Nähfaden verbunden ist, (i) einen distalen Abschnitt (5c), der eine solche Breite aufweist, dass er zumindest einen großen Teil der hinteren Hälfte der Wand der Vagina (2) abdecken und sehr seitlich, also in einer Weise, die die Paravagina nicht durchbohrt, vernäht werden kann, und (ii) einen abgerundeten Ausschnitt (5d) auf weist, der an seinen Seitenrand des distalen Endes (5e) eingebracht ist, wobei der Ausschnitt (5d) solche Abmessungen aufweist, die das Anordnen des stücks (5) um die Basis der Wand der Vagina (2) an zumindest einem großen Teil der hinteren Hälfte dieser Wand ermöglichen und dabei das Rektum in ausreichendem Abstand belassen, um nicht eine Kompression desselben zu riskieren; wobei der Nähfaden (6) mit dem Stück (5) bezüglich dem Ausschnitt (5d) seitlich versetzt verbunden ist.

2. Vorrichtung nach Anspruch 1, **dadurch gekennzeichnet, dass** sie eine zweite Anordnung (10) aufweist, die aus einem Stück (15) aus einem durchbrochenen Material, einem Nähfaden (16) und einer Nähnadel (17) gebildet wird, und die dazu bestimmt ist, in Ergänzung der ersten Anordnung (1) auf Höhe der Vorderseite der Vagina (2) an Ort und Stelle gebracht zu werden; wobei die zweite Anordnung ein längliches Stück (15) aus einem f lexiblen durchbrochenen Material, einen Nähfaden (16), der mit einem Längsende des Stücks (15) verbunden ist, und eine Nähnadel (17) aufweist, die mit dem Faden (16) verbunden ist; wobei das Stück (15) eine solche Länge, die eine vordere Suspension der Vagina (2) am Promontorium (3) ermöglicht, und auf der Seite des Endes (15e), das mit dem Faden (16) verbunden ist, zwei schräge Seitenränder (20) aufweist, die in Richtung dieses Endes (15e) derart zulaufen, dass das Stück (15) einen Abschnitt (15b) aufweist, der in Richtung des Endes (15e) zunehmend schmaler wird; wobei der Nähfaden (16) mit diesem Ende (15e) verbunden ist.

3. Vorrichtung nach Anspruch 2, **dadurch gekennzeichnet, dass** das Stück (15) aus durchbrochenem Material der zweiten Anordnung (10) ausgehend von seinem proximalen Ende in Längsrichtung geschlitzt ist, um zwei Stränge (15c) zu bilden, die dazu bestimmt sind, auf beiden Seiten der Gebärmutterenge an den Gebärmutteranhängen angeordnet und durch ihren proximalen Abschnitt am Promontorium (3) befestigt zu werden.

4. Vorrichtung nach einem der Ansprüche 1 bis 3, **dadurch gekennzeichnet, dass** jede Anordnung (1, 10) aus Stück aus durchbrochenem Material - Nähfaden - Nähnadel einen Nähfadenstrang (8, 18) aufweist, dessen eines Ende mit dem Stück (5, 15) aus durchbrochenem Material in unmittelbarer Nähe zum Ende des Nähfadens (6, 16) verbunden ist.

5. Vorrichtung nach Anspruch 4, **dadurch gekennzeichnet, dass** der Nähfaden (6, 16) durch Verknoten mit dem Stück (5, 15) aus durchbrochenem Material verbunden ist, und dass der Nähfadenstrang (8, 18) durch einen Abschnitt des Nähfadens (6, 16) gebildet ist, der sich über den Knoten (9) hinausgehend verlängert.

6. Vorrichtung nach einem der Ansprüche 1 bis 5, **dadurch gekennzeichnet, dass** jedes Stück (5, 15) aus durchbrochenem Material aus einem Gewirk aus vielfaserigen Polyesterfäden mit quadratischen Maschen hergestellt ist.

7. Vorrichtung nach einem der Ansprüche 1 bis 6, **dadurch gekennzeichnet, dass** jedes Stück (5, 15) mit Collagen getränkt ist.

8. Vorrichtung nach einem der Ansprüche 1 bis 7, **dadurch gekennzeichnet, dass** der Nähfaden (6, 16) durch ein Geflecht aus Polyesterfäden gebildet wird.

9. Vorrichtung nach einem der Ansprüche 1 bis 8, **dadurch gekennzeichnet, dass** der Nähfaden (6, 16) eine Länge in der Größenordnung von 25 cm aufweist.

10. Vorrichtung nach einem der Ansprüche 1 bis 9, **dadurch gekennzeichnet, dass** die Nähnadel (7, 17) eine ausreichend gekrümmte Form, um bei ihrer Einführung in die Scheidenwand diese nicht zu durchbohren, und einen dreieckigen Abschnitt aufweist, um ihren Durchgang durch mitunter feste Gewebe zu erleichtern.
